# EUROPÄISCHE PATENTSCHRIFT

(11) **EP 4 277 632 B1**
(45) Veröffentlichungstag und Bekanntmachung des Hinweises auf die Patenterteilung: **20.08.2025**
(21) Anmeldenummer: 22701561.7
(22) Anmeldetag: 14.01.2022
(51) Int. Cl.: A61K 31/522, A61K 9/00, A61K 47/32, A61K 47/10, A61K 47/14, A61K 47/26, A61K 47/44, A61K 47/36, A61K 47/38, A61K 31/19

(54) **ORALE DÜNNFILME**
ORAL THIN FILMS
FILMS ORODISPERSIBLES

(30) Priorität: 15.01.2021 DE 102021100718
(43) Veröffentlichungstag der Anmeldung: 22.11.2023
(73) Patentinhaber: LTS Lohmann Therapie-Systeme AG, 56626 Andernach (DE)
(72) Erfinder: MÜLLER, Markus, 53840 Troisdorf (DE); FICKER, Mario, 53179 Bonn (DE); LINN, Michael, 55596 Waldböckelheim (DE)
(74) Vertreter: Meissner Bolte Partnerschaft mbB
(86) Internationale Anmeldenummer: PCT/EP2022/050790
(87) Internationale Veröffentlichungsnummer: WO 2022/152876

(56) Entgegenhaltungen:
- WO-A1-2007/102817
- WO-A1-2015/195605
- US-A1- 2005 136 096
- US-A1- 2014 335 153
- US-A1- 2020 146 997

## Beschreibung

Die vorliegende Erfindung betrifft, ein Verfahren zur Herstellung eines mehrschichtigen oralen Dünnfilms, einen oralen Dünnfilm erhältlich durch dieses Verfahren und dessen Verwendung als Arzneimittel.

Orale Dünnfilme (OTF) sind dünne, in der Regel mindestens einen pharmazeutisch aktiven Wirkstoff enthaltende Filme, die direkt in den Mundraum gelegt oder an die Mundschleimhaut angelegt werden und sich dort auflösen. Diese dünnen oralen Dünnfilme können als ein- oder mehrschichtige Systeme aufgebaut sein. Der pharmazeutisch aktive Wirkstoff kann in dem Film gelöst, emulgiert oder dispergiert sein. Orale Dünnfilme sind jedoch nicht nur auf die Verabreichung von Arzneimitteln beschränkt, sondern finden auch Anwendung im Lebensmittelbereich, sowie in der Zahnpflege oder als Munderfrischer.

In verschiedenen Fällen kann es von Nöten sein, dass ein OTF eine lange Verweilzeit im Mund des Patienten aufweist. Durch permanenten Speichelfluss und Bewegungen im Mund unterliegen die Filme jedoch einer permanenten Erosion. Um dies zu verhindern, kann der OTF mit einer oder mehreren langsam löslichen oder unlöslichen Rückschicht(en) versehen werden.

Es könnte aber auch von Nöten sein, schnelllösliche Stoffe, wie z.B.

Geschmacksstoffe, Puffersubstanzen, Salze oder Süßstoffe als Rückschicht(en) auf das OTF aufzutragen.

Bei den aus dem Stand der Technik bekannten mehrschichtigen OTFs werden diese Rückschichten durch eine Doppel- bzw. Mehrfachbeschichtung aufgebracht oder mittels einer Klebeschicht aufgeklebt. Manche Zusammensetzungen für orale Dünnfilme werden in US 2005/136096 A1, US 2014/335153 A1, WO 2007/102817 A1, US 2020/146997 A1 und WO 2015/195605 A1 offenbart.

Ein Nachteil bei diesen bekannten Verfahren ist es, dass die Rückschicht separat hergestellt werden muss und dann mit dem eigentlichen Film durch Doppelbeschichtung verbunden oder über eine Klebeschicht aufgeklebt werden muss. Eine Doppel- bzw. Mehrfachbeschichtung führt zu erhöhtem thermischen Stress, insbesondere für den enthaltenen pharmazeutisch aktiven Wirkstoff, und führt zudem oft zu schlecht haftenden Verbünden. Durch die Diffusion des Lösungsmittels in den unteren Film bei der Doppel- bzw. Mehrfachbeschichtung muss das Lösungsmittel oft aufwendig wieder herausgetrocknet werden.

Auch für das Verkleben der verschiedenen Schichten ist es notwendig die Rückschicht separat herzustellen und anschließend zu verkleben. Vor allem bei Rücksichten, die sich bei der Applikation langsam auflösen sollen, müssen Polymere gewählt werden, die neben ihrer schlechteren Löslichkeit auch eine hohe Viskosität in der Polymerlösung mit sich bringen. Dies führt zu niedrigen Feststoffgehalten und somit zu langen Trocknungszeiten. Des Weiteren müssen die Filme damit sie nicht als störend empfunden werden sehr dünn sein, wodurch sie filigran und schwer handhabbar sind.

Die Aufgabe der vorliegenden Erfindung besteht darin, vorstehend genannte Nachteile des Standes der Technik zu beheben. Insbesondere besteht die Aufgabe der vorliegenden Erfindung darin, ein Verfahren zur Herstellung eines mehrschichtigen oralen Dünnfilms bereitzustellen, bei dem die einzelnen Schichten nicht separat hergestellt werden müssen und nicht durch Auflaminieren von einzelnen separat getrockneten Schichten zu einem Verbund zusammengefügt werden müssen. Ferner soll ein mehrschichtiger oraler Dünnfilm bereitgestellt werden, wobei die einzelnen Schichten vorzugsweise auch nicht durch eine separate Klebeschicht miteinander verbunden sind. Außerdem soll eine thermische Belastung des enthaltenen pharmazeutisch aktiven Wirkstoffs möglichst weitgehend vermieden werden.

Obige Aufgabe wird durch ein Verfahren zur Herstellung eines mehrschichtigen, vorzugsweise zweischichtigen, oralen Dünnfilms nach Anspruch 1 gelöst, d.h. durch ein Verfahren zur Herstellung eines mehrschichtigen oralen Dünnfilms umfassend die Schritte
a) Bereitstellen einer Lösung, einer Emulsion, eines Schaums oder einer Suspension umfassend mindestens ein erstes Polymer und mindestens einen Weichmacher,
b) Verarbeiten der Lösung, der Emulsion, des Schaums oder der Suspension aus Schritt a) um einen Polymerfilm zu erhalten, wobei die Menge des mindestens einen Weichmacher 0,1 bis 50 Gew.-%, bezogen auf das Gesamtgewicht des Polymerfilms, beträgt,
c) Aufbringen eines Pulvers umfassend mindestens einen zweiten, vom ersten Polymer verschiedenen Stoff, auf den Polymerfilm, wobei der mindestens eine zweite Stoff Polyethylenoxid, mit einem mittleren Molekulargewicht von 7.000.000 g/mol, umfasst, end
d) Bilden eines Verbundes zwischen dem Polymerfilm und dem in Schritt c) aufgebrachten Pulver.

Vorzugsweise wird das Pulver umfassend mindestens ein zweites vom ersten unterschiedliches Polymer, in Schritt c) auf den Polymerfilm aufgetragen, bevor dieser vollständig getrocknet ist.

Das in Schritt c) aufgetragenen Pulver umfasst ein zweites Polymer, wobei das zweite Polymer vom ersten Polymer verschieden ist.

Das in Schritt c) aufgetragene Polymer kann auch nicht polymere Stoffe umfassen, wie beispielsweise Süßstoffe, Geschmacksstoffe, Puffersubstanzen, Salze oder auch pharmazeutisch aktive Wirkstoffe.

Ein Vorteil dieses Verfahrens besteht darin, dass hier keine separate Herstellung verschiedener Schichten nötig ist. Es wird lediglich ein erster Polymerfilm hergestellt, auf den ein zweiter Stoff, vorzugsweise ein zweites Polymer, aufgetragen wird, bevorzugt bevor der erste Polymerfilm vollständig getrocknet ist.

Ein solcher, nicht vollständig getrockneter Polymerfilm zeichnet sich vorzugsweise dadurch aus, dass er eine gewisse Klebrigkeit aufweist, die dazu führt, dass der zweite Stoff, umfassend das zweite Polymer, auf dem Polymerfilm haftet und einen Verbund bildet. Durch anschließendes Trocknen des Verbundes kann somit ein mehrschichtiger oraler Dünnfilm bereitgestellt werden. Somit kann ein mehrschichtiger oraler Dünnfilm bereitgestellt werden, wobei die einzelnen Schichten nicht separat hergestellt und durch Verkleben oder Laminieren miteinander verbunden werden müssen. Zudem wird der mehrschichtige Orale Dünnfilm nicht übermäßig thermisch belastet.

Unter einem nicht vollständig getrockneten Polymerfilm wird ein Polymerfilm verstanden, der einen Restgehalt an Lösungsmittel von 0,1 bis 10 Gew.-% aufweist.

Sollte der Restlösungsmittelgehalt in dem Film zu niedrig sein, kann der Film zusätzlich mit Lösungsmittel besprüht werden, damit eine ausreichende Feuchtigkeit auf der Oberfläche des Films erzielt wird.

Vorzugsweise wird der Polymerfilm vor dem Aufbringen des zweiten Stoffs jedoch nicht weiter behandelt, insbesondere nicht durch das Aufbringen einer weiteren Substanz, die das Anhaften des zweiten Stoffs begünstigt, wie beispielsweise ein Klebstoff.

Das Verarbeiten der Lösung, der Emulsion des Schaums oder der Suspension erfolgt mit den üblichen Methoden, wie Ausstreichen, Extrudieren oder Drucken.

Das erfindungsgemäße Verfahren zeichnet sich ferner vorzugsweise dadurch aus, dass das Bilden des Verbundes ein Trocknen des Verbundes durch das Erwärmen des zweischichtigen Verbundes auf eine Temperatur oberhalb der Schmelztemperatur des ersten und/oder des zweiten Stoffes, vorzugsweise des zweiten Polymers umfasst.

Der Verbund muss nicht zwingend getrocknet (im Sinne von Lösungsmittel entfernen) werden. Es kann auch ein Erhitzen bzw. Aufschmelzen des aufgestreuten zweiten Stoffs, vorzugsweise des zweiten Polymers, oder ein Erreichen der Glasübergangstemperatur ausreichend sein. Auch ein Anpressen des aufgestreuten zweiten Stoffes, vorzugsweise des zweiten Polymers ist denkbar.

Dies hat den Vorteil, dass dadurch mindestens einer bzw. eines der mindestens zwei verschiedenen Stoffe bzw. Polymere erweicht bzw. schmilzt und es somit zu einem stabileren Verbund der Schichten kommt.

In einer alternativen Ausführungsform des erfindungsgemäßen Verfahrens kann es auch ausreichend sein, den Verbund auf eine Temperatur unterhalb des Schmelzpunktes der verwendeten Polymere zu erwärmen, um bereits eine Stabilisierung des Verbundes zu erreichen. Geeignete Temperaturen betragen beispielsweise von etwa 50°C bis etwa 120°C, vorzugsweise bis etwa 100°C, besonders bevorzugt bis etwa 80°C. Dies ist bevorzugt, wenn der Schmelzpunkt des ersten bzw. zweiten Stoffs, vorzugsweise des Polymers so hoch ist, dass eine Erwärmung auf eine Temperatur oberhalb dieser Schmelzpunkte die weiteren Inhaltsstoffe des mehrschichtigen oralen Dünnfilms, insbesondere einen eventuell vorhandenen pharmazeutisch aktiven Wirkstoff, beeinträchtigt.

Das erfindungsgemäße Verfahren zeichnet sich ferner vorzugsweise dadurch aus, dass der Polymerfilm beim Aufbringen des zweiten Stoffs, umfassend des mindestens einen zweiten Polymers, eine Restfeuchte von etwa 0,1 bis 10 Gew.- % aufweist. Unter Restfeuchte wird der Gehalt an Lösungsmittel in Gew.-% verstanden.

Ist weniger Restfeuchte in dem Polymerfilm vorhanden, dann haftet der zweite Stoff, vorzugsweise das mindestens eine zweite, vom ersten verschiedene, Polymer nicht mehr gut genug auf dem Polymerfilm.

Es ist ferner bevorzugt, dass der Polymerfilm eine gewisse Klebrigkeit, aufweist.

Klebrig ist ein Film dann, wenn er als Klebstoff bzw. Haftklebstoff, wie in der DIN EN 923:2016-03 definiert, wirken kann. Dies hilft dem mindestens einen zweiten, vom ersten verschiedene Polymer, auf der Oberfläche des Polymerfilms zu haften.

Das erfindungsgemäße Verfahren zeichnet sich dadurch aus, dass das mindestens eine zweite Polymer als Pulver aufgetragen wird. Das Pulver ist bevorzugt trocken, d.h. es weist eine Restfeuchte, wie oben definiert, von kleiner 10 Gew.-% auf.

Vorzugsweise weist dieses Pulver, das vorzugsweise ein trockenes Pulver ist, eine mittlere Partikelgröße von kleiner als 500µm, bevorzugt von kleiner als 200µm, ermittelt durch Dynamische Lichtstreuung, auf.

Das erfindungsgemäße Verfahren zeichnet sich ferner vorzugsweise dadurch aus, dass das mindestens eine erste Polymer ein wasserlösliches und/oder wasserquellbares Polymer umfasst.

Wasserlösliche Polymere umfassen chemisch sehr unterschiedliche, natürliche oder synthetische Polymere, deren gemeinsames Merkmal ihre Löslichkeit in Wasser oder wässrigen Medien ist. Voraussetzung dafür ist, dass diese Polymere eine für die Wasserlöslichkeit ausreichende Anzahl an hydrophilen Gruppen besitzen und nicht vernetzt sind. Die hydrophilen Gruppen können nicht-ionisch, anionisch, kationisch und/oder zwitterionisch sein.

Wasserquellbare Polymere sind Polymere, die durch das Eindringen von Wasser ihr Volumen vergrößern.

Das erfindungsgemäße Verfahren zeichnet sich ferner vorzugsweise dadurch aus, dass das mindestens eine erste Polymer ausgewählt ist aus Stärke und Stärkederivaten, Dextranen, Cellulosederivaten, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylaten, Polyvinylpyrrolidonen, Polyvinylalkoholen, Polyethylenoxidpolymeren, Polyacrylamiden, Polyethylenglycolen, Gelatine, Collagen, Alginaten, Pektin, Pullulan, Traganth, Chitosan, Alginsäure, Arabinogalaktan, Galaktomannan, Agar, Agarose, Carrageen und natürlichen Gummen.

Diese Polymere haben den Vorteil, dass sie mit einer Vielzahl von pharmazeutisch aktiven Wirkstoffen kompatibel und zudem weitgehend unbedenklich für einen Patienten sind, zu dessen Behandlung der erfindungsgemäße mehrschichtige orale Dünnfilm eingesetzt wird.

Diese Polymere haben außerdem den Vorteil, dass sie getrocknet einen dünnen stabilen Film bilden, der sich bei Applikation auf der Schleimhaut oder im Mundraum auflöst und damit den Wirkstoff freigibt. Dies hat den Vorteil einer schnellen Verfügbarkeit des Wirkstoffs sowie einer rückstandlosen Darreichung des Wirkstoffes.

Das erfindungsgemäße Verfahren ist außerdem vorzugsweise dadurch gekennzeichnet, dass das mindestens eine zweite Polymer ein wasserunlösliches Polymer umfasst.

Das erfindungsgemäße Verfahren kann außerdem vorzugsweise dadurch gekennzeichnet sein, dass das mindestens eine zweite Polymer ein langsam wasserlösliches Polymer oder ein wasserlösliches Polymer umfasst. Wasserlösliche Polymere umfassen chemisch sehr unterschiedliche, natürliche oder synthetische Polymere, deren gemeinsames Merkmal ihre Löslichkeit in Wasser oder wässrigen Medien ist. Voraussetzung ist, dass diese Polymere eine für die Wasserlöslichkeit ausreichende Anzahl an hydrophilen Gruppen besitzen und nicht vernetzt sind. Die hydrophilen Gruppen können nicht-ionisch, anionisch, kationisch und/oder zwitterionisch sein.

Unter wasserlöslich wird vorzugsweise eine Löslichkeit von größer als 100 g/L in Wasser bei 25°C verstanden.

Das mindestens eine zweite Polymer Polyethylenoxid umfasst, mit einem mittleren Molekulargewicht von etwa 7.000.000 g/mol. Der Einsatz eines solchen wasserunlöslichen bzw. schwer wasserlöslichen Polymers hat den Vorteil, dass auf einer Seite des mehrschichtigen oralen Dünnfilms eine Schicht gebildet wird, die den mehrschichtigen oralen Dünnfilm vor Erosion bzw. schneller Auflösung schützt.

Das erfindungsgemäße Verfahren ist außerdem dadurch gekennzeichnet, dass der Polymerfilm, neben dem mindestens einen ersten Polymer zusätzlich mindestens einen Weichmacher enthält.

Weichmacher sind flüssige oder feste, indifferente organische Substanzen, vorzugsweise mit geringem Dampfdruck, welche ohne chemische Reaktion, vorzugsweise durch ihr Löse- und Quellvermögen, unter Umständen aber auch ohne ein solches, mit hochpolymeren Stoffen in physikalische Wechselwirkung treten und mit diesen ein homogenes System bilden können. Weichmacher verleihen den mit ihnen hergestellten Gebilden bzw. Überzügen bestimmte angestrebte physikalische Eigenschaften, wie z. B. erniedrigte Einfriertemperatur, erniedrigte Glasübergangstemperatur, erhöhtes Formveränderungsvermögen, erhöhte elastische Eigenschaften, verringerte Härte und gegebenenfalls gesteigertes Haftvermögen. Sie gehören zu den Kunststoffadditiven.

Es ist bevorzugt, dass der mindestens eine Weichmacher Glycerin, Triacetin, Polyethylenglykol, insbesondere Polyethylenglykol 200, Sorbitol, Wasser, Ethanol und/oder Tributylcitrat umfasst.

Dies ist insbesondere vorteilhaft, da viele wasserlösliche Polymere durch den Zusatz von Weichmachern klebrig gemacht werden können.

Da ein Weichmacher in dem Polymerfilm vorhanden ist, so ist es bevorzugt, dass die Menge des mindestens einen ersten Polymers in dem Polymerfilm etwa 25 bis 90 Gew.-%, bevorzugt etwa 35 bis 85 Gew.-%, besonders bevorzugt etwa 40 bis 80 Gew.-%, bezogen auf das Gesamtgewicht des Polymerfilms, beträgt.

Die Menge des mindestens einen Weichmachers in dem Polymerfilm 0,1 bis 50 Gew.-% beträgt, bevorzugt etwa 0,5 bis 25 Gew.-%, bezogen auf das Gesamtgewicht des Polymerfilms.

Wird zu wenig oder zu viel Weichmacher eingesetzt, so ist die Mischung entweder nicht klebrig oder ein verarbeitbarer Masseansatz kann von vorneherein nicht bereitgestellt werden.

Der Einsatz eines Polymerfilms, der durch den Zusatz von mindestens einem Weichmacher klebrig gemacht wurde, hat den Vorteil, dass der zweite Stoff, umfassend das zweite Polymer, dass auf den Polymerfilm aufgebracht wird, besonders gut haftet.

Besonders bevorzugt ist eine Mischung aus Polyvinylalkohol und/oder Polyvinylpyrrolidon als erstes wasserlösliches Polymer und Glycerin als Weichmacher, vorzugsweise in einem Verhältnis von etwa 80:20 Gew.-%

Das erfindungsgemäße Verfahren ist außerdem vorzugsweise dadurch gekennzeichnet, dass der Polymerfilm mindestens einen pharmazeutisch aktiven Wirkstoff umfasst, und/oder wobei mindestens ein pharmazeutisch aktiver Wirkstoff entweder alleine oder zusammen mit dem mindestens einen zweiten Polymer auf den Polymerfilm aufgebracht wird.

Ist der mindestens eine pharmazeutisch aktive Wirkstoff in dem Polymerfilm enthalten, so liegt der mindestens eine pharmazeutisch aktive Wirkstoff vorzugsweise in diesem gelöst, emulgiert bzw. suspendiert vor.

Wird der mindestens eine pharmazeutisch aktive Wirkstoff alleine oder zusammen mit dem mindestens einen zweiten Polymer auf den Polymerfilm aufgebracht, so wird dieser bevorzugt in Pulverform aufgebracht. Der mindestens eine pharmazeutisch aktive Wirkstoff liegt bevorzugt als trockenes Pulver vor, d.h. er weist eine Restfeucht, wie oben definiert, von kleiner als 10 Gew.-% auf.

Vorzugsweise weist der pulverförmige mindestens eine pharmazeutisch aktive Wirkstoff eine mittlere Partikelgröße von kleiner als 500 µm, bevorzugt von kleiner als 200 µm, gemessen mittels Dynamischer Lichtstreuung, auf.

Der mindestens eine pharmazeutisch aktive Wirkstoff ist nicht beschränkt, sondern umfasst alle pharmazeutisch aktiven Wirkstoffe, die mittels eines OTFs verabreichbar sind.

Geeignete pharmazeutisch aktive Wirkstoffe sind vorzugsweise ausgewählt aus der Gruppe, bestehend aus Analgetika, Hormonen, Hypnotika, Sedativa, Antiepileptika, Weckaminen, Psychoneurotropika, Neuro-Muskelblockern, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormonen, Sexualhormonen, Antidiabetika, Antitumor-Wirkstoffen, Antibiotika, Chemotherapeutika und Narcotika.

In einer weiteren alternativen Ausführungsform des mehrschichtigen oralen Dünnfilms, wird der mindestens eine pharmazeutisch aktive Wirkstoff als zweiter Stoff auf den Polymerfilm aufgetragen.

Alternativ wird eine Puffersubstand, ein Süßstoff und/oder ein Salz aufgetragen.

Das erfindungsgemäße Verfahren ist vorzugsweise dadurch gekennzeichnet, dass mindestens eine weitere Schicht, vorzugsweise eine weiteren Polymerschicht, auf der Seite des zweischichtigen Verbundes auf der nicht der zweite Stoff, vorzugsweise das zweite Polymer, aufgebracht wurde, aufgebracht wird.

Dies kann durch dem Fachmann bekannte, übliche Verfahren, wie Laminieren und/oder Verkleben erfolgen. Diese weitere mindestens eine Schicht kann gleiche und weitere pharmazeutisch aktive Wirkstoffe oder Puffersubstanzen, Süßstoffe, Geschmacksstoffe oder Salze umfassen. Ferner können in dieser spezielle mukoadhäsive Polymere eingesetzt werden, die die Haftung des mehrschichtigen oralen Dünnfilms auf der Schleimhaut des Patienten erhöhen.

Das erfindungsgemäße Verfahren ist vorzugsweise dadurch gekennzeichnet, dass der Polymerfilm zusätzlich mindestens einen Hilfsstoff ausgewählt aus der Gruppe, umfassend Farbstoffe, Aromastoffe, Süßstoffe, geschmacksmaskierende Mittel, Tenside, Enhancer, pH-Regulatoren, Konservierungsmittel und/oder Antioxidantien enthält und/oder mindestens einer dieser Hilfsstoffe zusammen mit dem mindestens einen zweiten Polymer auf den Polymerfilm aufgebracht wird.

Wird der zusätzlich mindestens eine Hilfsstoff zusammen mit dem zweiten Stoff, vorzugsweise dem zweiten Polymer auf den Polymerfilm aufgebracht, so wird dieser bevorzugt in Pulverform aufgebracht. Der mindestens eine Hilfsstoff liegt bevorzugt als trockenes Pulver vor, d.h. er weist eine Restfeucht, wie oben definiert, von kleiner 10 Gew.-% auf.

Es ist ferner möglich als zweiten Stoff einen Superabsorber alleine oder zusammen mit dem mindestens einen zweiten Stoff auf den Polymerfilm aufzubringen. Dies ermöglicht es auch Flüssigkeiten, die durch den Superabsorber absorbiert werden, zu applizieren.

Das erfindungsgemäße Verfahren ist außerdem vorzugsweise dadurch gekennzeichnet, dass der mehrschichtige orale Dünnfilm ein Flächengewicht von 50 bis 400 g/m² aufweist.

Die vorliegende Erfindung betrifft ferner einen mehrschichtigen oralen Dünnfilm erhältlich durch das vorstehend beschriebene Verfahren.

Die vorliegende Erfindung betrifft weiterhin einen mehrschichtigen oralen Dünnfilm erhältlich nach dem vorstehend geschilderten Verfahren zur Verwendung als Arzneimittel.

Die Erfindung wird nachfolgend anhand von nicht beschränkenden Beispielen erläutert.

### Beispiele

### Beispiel 1

Auf einen silikonisierten Beschichtungsträger wurde ein Polymerfilm gemäß Rezeptur 1 aufgetragen. Das verwendete Polymer, Polyvinylpyrrolidon, hat die Eigenschaft, dass es mit dem Weichmacher Glycerin versehen auch klebrig ist. Unmittelbar nach dem Ausstreichen wurde die Oberfläche mit fein gesiebtem (<160 µm) Polyethylenoxid (Polyox VVSR-303; Molekulargewicht 7.000.000) abgestreut. Das überschüssige pulverförmige Polymer wurde mit einem Pinsel abgebürstet und der Film anschließend getrocknet. Zum Ende wurde der Film 5 min auf eine Temperatur oberhalb des Schmelzpunktes beider Polymere erhitzt. Der hierdurch resultierende Verbund konnte leicht von dem silikonisierten Beschichtungsträger abgelöst und weiterverarbeitet werden. Der Verbund kann, falls gewünscht, auf ein weiteres Laminat aufgewalzt werden. So entsteht ein Verbund der auf einer Seite eine mukoadhäsive Schicht aufweist und auf der anderen Seite eine Schicht, die den Film von Erosion schützt.

**Tabelle 1 mit Rezeptur 1:**

| Material | Anteil (Gew.-% trocken) | Beschreibung |
|---|---|---|
| PVP VA64 | 80 | Polymer |
| Glycerin | 20 | Weichmacher |

### Referenzbeispiel 2

Im Folgenden werden drei verschiedene Kategorien zur Darstellung der Erfindung aufgezeigt.
1. Beispiele, wobei Kollicoat MAE 100 als Pulver direkt auf eine Masse aufgestreut und dann getrocknet wird.
2. Beispiele mit Klebeschicht, wobei eine Klebeschicht hergestellt und auf ein vorab bereitgestelltes Schaumlaminat auflaminiert wurde. Anschließend wurden die pulverförmigen Materialien aufgestreut, abgedeckt und mit einer Walze angedrückt.
3. Beispiele mit Polyox, wobei ein Polyox-Laminat hergestellt, Pulver aufgetragen und anschließend über Schmelzpunkt von Polyox erhitzt wurde.

### Kategorie 1:

Herstellung des Basisfilms + Pulverauftrag:
Die Masse gemäß der folgenden Tabelle 2 wurde hergestellt und die pulverförmigen Materialien sofort aufgestreut. Anschließend wurde im Trockenschrank getrocknet.

**Tabelle 2:**

| Zusammensetzung: | | | |
|---|---|---|---|
| Ausgangsstoff | Funktion | Anteil mg/m² | Anteil % |
| Kollicoat MAE100-55 | Polymer | 9,742 | 20,95 |
| Kollicoat MAE 100 P | Polymer | 29,272 | 62,95 |
| Triethylcitrat | Weichmacher | 4,650 | 10 |
| Cherry US | Aroma | 1,395 | 3 |
| Saccharin NA | Süßungsmittel | 0,930 | 2 |
| Sucralose | Süßungsmittel | 0,465 | 1 |
| Menthol | Aroma | 0,047 | 0,1 |
| | | | |
| | Summe | 46,5 | 100 |

Die Gewichtsbestimmung erfolgte anhand eines 10 cm² Prüflings.
Beschichtungsdicke: 150 µm
Beschichtungsträger: 75 µm PET AB1 auf AB
Trocknung: 15 min bei 70°C
Flächengewicht: 46,5 cm/m²

Es wurden 9 verschiedene pulverförmigen Materialien gemäß der folgenden Tabelle 3 aufgestreut.

**Tabelle 3:**

| | | Pulver | | | |
|---|---|---|---|---|---|
| Basisfilm | Technik | Funktion | Material | Gesamtgewicht [g/m²] | Gewicht Pulver [g/m²] |
| Kollicoat MAE 100 Film Flächengewicht 46,5 [g/m²] | Aufgestreut vor Trocknen | Superabsorber | Kelkogel | 75,8 | 29,30 |
| | | Ionentauscher | Amberlite IRP64 | 75,8 | 29,30 |
| | | Aroma | Firmenich Peach | 93,7 | 47,20 |
| | | Masking | Mane Bitter Masking | 98,1 | 51,60 |
| | | Puffer | Trinatriumcitra t | 417,1 | 370,60 |
| | | Süßungsmittel | Sucralose | 66,9 | 20,40 |
| | | Polymer (mucoadhäsiv) | Kollidon VA 64 | 108,8 | 62,30 |
| | | API | Coffein | 77 | 30,50 |
| | | API Gemisch | Gemisch zum Aufschmelzen | 143,4 | 96,90 |

### Kategorie 2:

Herstellung eines Klebelaminats + Pulverauftrag:
Eine Klebeschicht wurde hergestellt und auf ein vorab bereitgestelltes Schaumlaminat auflaminiert. Die Zusammensetzung der Klebeschicht und des Schaumlaminats sind in Tabelle 4 zusammengefasst. Anschließend wurden die pulverförmigen Materialien aufgestreut, abgedeckt und mit einer Walze angedrückt. Die Probenentnahme erfolgte analog zu Kategorie 1.

**Tabelle 4:**

| | Zusammensetzung | | | |
|---|---|---|---|---|
| | Ausgangsstoff | Funktion | Anteil mg/m² | Anteil % |
| Schaumlaminat* | PVA 4-88 | Polymer | 77,60 | 52,33 |
| 100 g/m² | FD&C rot nr 40 | Farbstoff | 0,40 | 0,27 |
| | Saccharin NA | Süßungsmittel | 2,00 | 1,35 |
| | Sucralose | Süßungsmittel | 4,00 | 2,70 |
| | Glycerin | Weichmacher | 9,00 | 6,07 |
| | Cherry US | Aroma | 6,00 | 4,05 |
| | Menthol | Aroma | 1,00 | 0,67 |
| | | | | |
| Klebeschicht: | Vinylalkohol (VA64) | Polymer | 38,64 | 26,06 |
| 48,3 g/m² | Glycerin | Weichmacher | 9,66 | 6,51 |
| | | | | |
| | | Summe | 148,3 | 100,00 |

| | | | | |
|---|---|---|---|---|
| *Geschäumt mit Umgebungsluft, alternativ ist Schäumen mit Stickstoff möglich. Beschichtungsdicke: 150 µm Beschichtungsträger: 75 µm PET AB1 auf AB Trocknung: 10 min bei RT und 8 min bei 70°C Flächengewicht: 148,3 cm/m² | | | | |

Es wurden 8 verschiedene pulverförmigen Materialien gemäß der folgenden Tabelle 5 aufgestreut.

**Tabelle 5:**

| | | Pulver | | | |
|---|---|---|---|---|---|
| Basisfilm | Technik | Funktion | Material | Gesamtge wicht [g/m²] | Gewicht Pulver [g/m²] |
| Schaum+ Klebeschicht Flächengewicht 148,3 g/cm² | Auf Klebeschicht angedrückt | Superabsorber | Kelkogel | 161,9 | 13,60 |
| | | Ionentauscher | Amberlite IRP64 | 155,1 | 6,80 |
| | | Aroma | Firmenich Peach | 159,1 | 10,80 |
| | | Masking | Mane Bitter Masking | 173,4 | 25,10 |
| | | Puffer | Trinatriumcitrat | 403,2 | 254,90 |
| | | Polymer (mucoadhäsiv) | Kollidon VA 64 | 164,1 | 15,80 |
| | | API | Coffein | 149,1 | 0,80 |
| | | API Gemisch | Gemisch zum Aufschmelzen | 154 | 5,70 |

### Kategorie 3:

Herstellung eines Polyox-Laminats + Pulverauftrag:
Die Masse gemäß Tabelle 6 wurde beschichtet und getrocknet. Nach dem Trocknen wurden die pulverförmigen Materialen aufgestreut, die Striche abgedeckt, auf ein Blech gelegt und mit einem weiteren Blech von oben beschwert. Im Anschluss wurden die Striche zwischen den Blechen in den Trockenschrank eingelegt und bei 120°C aufgeschmolzen. Nach dem Abkühlen erfolget die Probenentnahme analog zu Kategorie 1.

**Tabelle 6:**

| Zusammensetzung | | | |
|---|---|---|---|
| Ausgangsstoff | Funktion | Anteil mg/m² | Anteil % |
| Polyox | Polymer | 92,63 | 97,5 |
| Glycerol | Weichmacher | 2,19 | 2,3 |
| FD&C rot nr 40 | Farbstoff | 0,19 | 0,2 |
| | | | |
| | Summe | 95,00 | 100 |

| | | | |
|---|---|---|---|
| Beschichtungsdicke: 300 µm Beschichtungsträger: 100 µm PET TSP Trocknung: 15 min bei 70°C Flächengewicht: 95 cm/m² | | | |

Aufschmelzschritt:
Abdeckfolie: 75 µm PET AB1 auf AB
Aufschmelzen: 10 min bei 120°C

Es wurden 8 verschiedene pulverförmigen Materialien gemäß der folgenden Tabelle 7 aufgestreut.

**Tabelle 7:**

| | | Pulver | | | |
|---|---|---|---|---|---|
| Basisfilm | Technik | Funktion | Material | Gesamtgew icht [g/m²] | Gewicht Pulver [g/m²] |
| Polyox Flächengewicht 95 g/m² | Eingeschmolzen | Superabsorber | Kelkogel | 119,8 | 73,30 |
| | | Ionentauscher | Amberlite IRP64 | 94,2 | 47,70 |
| | | Aroma | Firmenich Peach | 168,4 | 121,90 |
| | | Masking | Mane Bitter Masking | 146 | 99,50 |
| | | Puffer | Trinatriumcitrat | 482,3 | 435,80 |
| | | Polymer (mucoadhäsiv) | Kollidon VA 64 | 159,9 | 113,40 |
| | | API | Coffein | 196,7 | 150,20 |
| | | API Gemisch | Gemisch zum Aufschmelzen | 96,6 | 50,10 |

## Patentansprüche

1. Verfahren zur Herstellung eines mehrschichtigen oralen Dünnfilms umfassend die Schritte
a) Bereitstellen einer Lösung, einer Emulsion, eines Schaums oder einer Suspension umfassend mindestens ein erstes Polymer und mindestens einen Weichmacher,
b) Verarbeiten der Lösung, der Emulsion, des Schaums oder der Suspension aus Schritt a) um einen Polymerfilm zu erhalten, wobei die Menge des mindestens einen Weichmacher 0,1 bis 50 Gew.-%, bezogen auf das Gesamtgewicht des Polymerfilms, beträgt,
c) Aufbringen eines Pulvers umfassend mindestens einen zweiten, vom ersten Polymer verschiedenen Stoff, auf den Polymerfilm, wobei der mindestens eine zweite Stoff Polyethylenoxid, mit einem mittleren Molekulargewicht von 7.000.000 g/mol, umfasst, und
d) Bilden eines Verbundes zwischen dem Polymerfilm und dem in Schritt c) aufgebrachten Pulver.

2. Verfahren gemäß Anspruch 1, wobei das Bilden des Verbundes das Erwärmen des Verbundes auf eine Temperatur oberhalb der Schmelztemperatur des ersten und/oder des zweiten Stoffes umfasst.

3. Verfahren gemäß irgendeinem der vorhergehenden Ansprüche, wobei der Polymerfilm beim Aufbringen des mindestens einen zweiten Stoffes eine Restfeuchte von 0,1% bis 10 Gew.-% aufweist.

4. Verfahren gemäß irgendeinem der vorhergehenden Ansprüche, wobei der mindestens eine zweite Stoff mindestens ein zweites vom ersten verschiedenes Polymer als trockenes Pulver, vorzugsweise mit einer Partikelgröße von kleiner 500 µm umfasst.

5. Verfahren gemäß irgendeinem der vorhergehenden Ansprüche, wobei das mindestens eine erste Polymer ein wasserlösliches oder wasserquellbares Polymer umfasst.

6. Verfahren gemäß irgendeinem der vorhergehenden Ansprüche, wobei das mindestens eine erste Polymer ausgewählt ist aus Stärke und Stärkederivaten, Dextranen, Cellulosederivaten, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylaten, Polyvinylpyrrolidonen, Polyvinylalkoholen, Polyethylenoxidpolymeren, Polyacrylamiden, Polyethylenglycolen, Gelatine, Collagen, Alginaten, Pektin, Pullulan, Traganth, Chitosan, Alginsäure, Arabinogalaktan, Galaktomannan, Agar, Agarose, Carrageen und/oder natürlichen Gummen.

7. Verfahren gemäß irgendeinem der vorhergehenden Ansprüche, wobei der Polymerfilm mindestens einen Weichmacher in einer Menge von 0,5 bis 25 Gew.- %, bezogen auf das Gesamtgewicht des Polymerfilms, enthält.

8. Verfahren gemäß irgendeinem der vorhergehenden Ansprüche, wobei der Polymerfilm mindestens einen pharmazeutisch aktiven Wirkstoff umfasst, und/oder wobei mindestens ein pharmazeutisch aktiver Wirkstoff als zweiter Stoff auf den Polymerfilm aufgebracht wird, wobei der pharmazeutisch aktive Wirkstoff vorzugsweise ausgewählt ist aus der Gruppe, bestehend aus Analgetika, Hormonen, Hypnotika, Sedativa, Antiepileptika, Weckaminen, Psychoneurotropika, Neuro-Muskelblockern, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormonen, Sexualhormonen, Antidiabetika, Antitumor-Wirkstoffen, Antibiotika, Chemotherapeutika und Narcotika.

9. Verfahren gemäß irgendeinem der vorhergehenden Ansprüche ferner umfassend das Aufbringen mindestens einer weiteren Schicht auf der Seite des Verbundes auf der nicht das zweite Polymer aufgebracht wurde.

10. Verfahren gemäß Anspruch 9, wobei die weitere Schicht eine Polymerschicht ist.

11. Verfahren gemäß irgendeinem der vorhergehenden Ansprüche, wobei der Polymerfilm zusätzlich mindestens einen Hilfsstoff ausgewählt aus der Gruppe, umfassend Farbstoffe, Aromastoffe, Süßstoffe, geschmacksmaskierende Mittel, Tenside, Enhancer, pH-Regulatoren, Konservierungsmittel und/oder Antioxidantien, enthält und/oder mindestens einer dieser Hilfsstoffe zusammen mit dem mindestens einen zweiten Polymer auf den Polymerfilm aufgebracht wird.

12. Verfahren gemäß irgendeinem der vorhergehenden Ansprüche, wobei der mehrschichtige orale Dünnfilm ein Flächengewicht von 50 bis 400 g/m² aufweist.

13. Mehrschichtiger oraler Dünnfilm erhältlich nach dem Verfahren gemäß irgendeinem der Ansprüche 1 bis 12.

14. Mehrschichtiger oraler Dünnfilm gemäß Anspruch 13 zur Verwendung als Arzneimittel.

## Claims

1. A method for producing a multi-layer oral thin film comprising the steps of
a) providing a solution, an emulsion, a foam or a suspension comprising at least one first polymer and at least one plasticiser,
b) processing the solution, the emulsion, the foam or the suspension from step a) in order to obtain a polymer film, wherein the amount of the at least one plasticiser is 0.1 to 50 wt.%, in relation to the total weight of the polymer film,
c) applying a powder comprising at least one second substance, to the polymer film, wherein the at least one second substance comprises polyethylene oxide, with an average molecular weight of 7,000,000 g/mol, and
d) forming a composite between the polymer film and the powder applied in step c).

2. The method according to claim 1, wherein the forming of the composite comprises the heating of the composite to a temperature above the melting point of the first and/or the second substance.

3. The method according to any one of the preceding claims, wherein the polymer film has a residual moisture of from 0.1% to 10 wt.% at the time of application of the at least one second substance.

4. The method according to any one of the preceding claims, wherein the at least one second substance comprises at least one second polymer, which differs from the first polymer, as dry powder, preferably with a particle size of less than 500 µm.

5. The method according to any one of the preceding claims, wherein the at least one first polymer comprises a water-soluble or water-swellable polymer.

6. The method according to any one of the preceding claims, wherein the at least one first polymer is selected from starch and starch derivatives, dextrans, cellulose derivatives, such as carboxymethyl cellulose, hydroxypropyl cellulose, hydroxyethyl cellulose, hydroxypropyl methylcellulose, hydroxypropyl ethyl cellulose, sodium carboxymethyl cellulose, ethyl or propyl cellulose, polyacrylic acids, polyacrylates, polyvinylpyrrolidones, polyvinyl alcohols, polyethylene oxide polymers, polyacrylamides, polyethylene glycols, gelatines, collagen, alginates, pectin, pullulan, tragacanth, chitosan, alginic acid, arabinogalactan, galactomannan, agar, agarose, carrageenan, and/or natural gums.

7. The method according to any one of the preceding claims, wherein the polymer film contains at least one plasticiser in an amount of from 0.5 to 25 wt.%, in relation to the total weight of the polymer film.

8. The method according to any one of the preceding claims, wherein the polymer film comprises at least one pharmaceutically active agent, and/or wherein at least one pharmaceutically agent is applied as second substance to the polymer film, wherein the pharmaceutically active agent is preferably selected from the group consisting of analgesics, hormones, hypnotics, sedatives, antiepiletics, analeptics, psychoneurotropic drugs, neuro-muscle blockers, antspasmodics, antihistamines, antiallergics, cardiotonics, antiarrhythmics, diuretics, hypotensives, vasopressors, antidepressants, antitussives, expectorants, thyroid hormones, sexual hormones, antidiabetics, antitumour active agents, antibiotics, chemotherapeutics and narcotics.

9. The method according to any one of the preceding claims, further comprising the application of at least one further layer to the side of the composite to which the second polymer has not been applied.

10. The method according to claim 9, wherein the further layer is a polymer layer.

11. The method according to any one of the preceding claims, wherein the polymer film additionally contains at least one auxiliary substance selected from the group comprising colouring agents, flavourings, sweeteners, taste-masking agents, surfactants, enhancers, pH regulators, preservatives and/or antioxidants, and/or at least one of these auxiliary substances is applied together with the at least one second polymer to the polymer film.

12. The method according to any one of the preceding claims, wherein the multi-layer oral thin film has an area density of from 50 to 400 g/m².

13. A multi-layer oral thin film obtainable by the method according to any one of claims 1 to 12.

14. The multi-layer oral thin film according to claim 13 for use as a medicament.

## Revendications

1. Procédé de fabrication d'un film mince oral multicouche comprenant les étapes
a) de fourniture d'une solution, d'une émulsion, d'une mousse ou d'une suspension comprenant au moins un premier polymère et au moins un plastifiant,
b) de traitement de la solution, de l'émulsion, de la mousse ou de la suspension issue de l'étape a) pour obtenir un film polymère, dans lequel la quantité de l'au moins un plastifiant va de 0,1 à 50 % en poids, par rapport au poids total du film polymère,
c) d'application d'une poudre, comprenant au moins une deuxième substance différente du premier polymère, sur le film de polymère, dans lequel l'au moins une deuxième substance comprend de l'oxyde de polyéthylène avec un poids moléculaire moyen de 7 000 000 g/mol, et
d) de formation d'un composite entre le film polymère et la poudre appliquée à l'étape c).

2. Procédé selon la revendication 1, dans lequel la formation du composite comprend le chauffage du composite à une température supérieure à la température de fusion de la première et/ou de la deuxième substance.

3. Procédé selon l'une quelconque des revendications précédentes, dans lequel le film polymère présente une humidité résiduelle de 0,1 % à 10 % en poids lors de l'application de l'au moins une deuxième substance.

4. Procédé selon l'une quelconque des revendications précédentes, dans lequel l'au moins une deuxième substance comprend au moins un deuxième polymère différent du premier polymère différent en tant que poudre sèche, de préférence avec une taille de particules inférieure à 500 µm.

5. Procédé selon l'une quelconque des revendications précédentes, dans lequel l'au moins un premier polymère comprend un polymère hydrosoluble ou pouvant gonfler dans l'eau.

6. Procédé selon l'une quelconque des revendications précédentes, dans lequel l'au moins un premier polymère est choisi parmi l'amidon et des dérivés d'amidon, des dextranes, des dérivés de cellulose, tels que carboxyméthylcellulose, hydroxypropylcellulose, hydroxyéthylcellulose, hydroxypropylméthylcellulose, hydroxypropyléthylcellulose, carboxyméthylcellulose de sodium, éthylcellulose ou propylcellulose, des acides polyacryliques, des polyacrylates, des polyvinylpyrrolidones, des alcools polyvinyliques, des polymères d'oxyde de polyéthylène, des polyacrylamides, des polyéthylène glycols, de la gélatine, du collagène, des alginates, de la pectine, du pullulan, de la gomme adragante, du chitosan, de l'acide alginique, de l'arabinogalactane, du galactomannane, de l'agar, de l'agarose, du carraghénane et/ou des gommes naturelles.

7. Procédé selon l'une quelconque des revendications précédentes, dans lequel le film polymère contient au moins un plastifiant en une quantité de 0,5 à 25 % en poids, par rapport au poids total du film polymère.

8. Procédé selon l'une quelconque des revendications précédentes, dans lequel le film polymère comprend au moins un actif pharmaceutiquement actif, et/ou dans lequel au moins un actif pharmaceutiquement actif est appliqué en tant que deuxième substance sur le film polymère, dans lequel l'actif pharmaceutiquement actif est de préférence choisi dans le groupe, composé d'analgésiques, d'hormones, d'hypnotiques, de sédatifs, d'antiépileptiques, des weckamines, de psychoneurotropes, de neurobloquants, d'antispasmodiques, d'antihistaminiques, d'antiallergiques, de cardiotoniques, d'antiarythmiques, de diurétiques, d'hypotenseurs, de vasopresseurs, d'antidépresseurs, d'antitussifs, d'expectorants, d'hormones thyroïdiennes, d'hormones sexuelles, d'antidiabétiques, d'agents antitumoraux, d'antibiotiques, d'agents chimiothérapeutiques et de narcotiques.

9. Procédé selon l'une quelconque des revendications précédentes comprenant en outre l'application d'au moins une couche supplémentaire du côté du composite sur lequel le deuxième polymère n'a pas été appliqué.

10. Procédé selon la revendication 9, dans lequel la couche supplémentaire est une couche polymère.

11. Procédé selon l'une quelconque des revendications précédentes, dans lequel le film polymère contient en supplément au moins un excipient choisi dans le groupe comprenant des colorants, des arômes, des édulcorants, des agents de masquage des arômes, des tensioactifs, des amplificateurs, des régulateurs de pH, des conservateurs et/ou des antioxydants, et/ou au moins un de ces excipients est appliqué conjointement avec l'au moins un deuxième polymère sur le film polymère.

12. Procédé selon l'une quelconque des revendications précédentes, dans lequel le film mince oral multicouche présente un poids surfacique de 50 à 400 g/m ².

13. Film mince oral multicouche pouvant être obtenu selon le procédé selon l'une quelconque des revendications 1 à 12.

14. Film mince oral multicouche selon la revendication 13 destiné à être utilisé en tant que médicament.
